# EUROPEAN PATENT SPECIFICATION

(11) **EP 2 582 797 B1**
(45) Date of publication and mention of the grant of the patent: **25.02.2015**
(21) Application number: 11745847.1
(22) Date of filing: 20.06.2011
(51) Int. Cl.: C12N 7/02, C07K 14/01

(54) **A METHOD OF PRODUCING BACTERIOPHAGES**
METHODE FÜR DIE PRODUKTION VON BAKTERIOPHAGEN
PROCÉDÉ DE PRODUCTION DE BACTÉRIOPHAGES

(30) Priority: 20.06.2010 PL 39155110
(43) Date of publication of application: 24.04.2013
(73) Proprietor: Instytut Immunologii I Terapii Doswiadczalnej Pan, 53-114 Wroclaw (PL)
(72) Inventor: DABROWSKA, Krystyna, 54-614 Wroclaw (PL); OSLIZLO, Anna, Nysa Nysa (PL); BUDYNEK, Paulina, 48-340 Glucholazy (PL); PIOTROWICZ, Agnieszka, 54-440 Wroclaw (PL); GÓRSKI, Andrzej, 50-540 Wroclaw (PL)
(74) Representative: Witek, Rafal
(86) International application number: PCT/PL2011/050026
(87) International publication number: WO 2011/162627

(56) References cited:
- US-A1- 2005 158 712
- US-A1- 2005 226 892
- JIANG J ET AL: "Display of a porA peptide from neisseria meningitidis on the bacteriophage T4 capsid surface", INFECTION AND IMMUNITY, vol. 65, no. 11, 1 January 1997 (1997-01-01), pages 4770-4777, XP002903999, ISSN: 0019-9567
- ANNA OSLIZLO ET AL: "Purification of phage display-modified bacteriophage T4 by affinity chromatography", BMC BIOTECHNOLOGY, vol. 11, no. 1, 1 January 2011 (2011-01-01), pages 59-59, XP55010029, ISSN: 1472-6750, DOI: 10.1186/1472-6750-11-59
- BORATYNSKI JANUSZ ET AL: "Preparation of endotoxin-free bacteriophages", CELLULAR AND MOLECULAR BIOLOGY LETTERS, vol. 9, no. 2, 1 January 2004 (2004-01-01) , pages 253-259, XP002485107, ISSN: 1425-8153 cited in the application

## Description

The present invention relates to a method of producing purified bacteriophage preparations. The resulting bacteriophage preparations are useful in many applications, particularly in the manufacturing of products requiring a high degree of purity, such as medicinal products.

During the lytic cycle during therapy, bacteria are destroyed by the bacteriophages multiplying in them. Bacteriophage progeny, are released in amplified numbers into the environment, and subsequently lyse the following bacterial populations. Regarding the production of bacteriophage lysates for technical purposes, not only the number of progeny phage molecules is significant, but the bacterial structural elements released into the environment as well, such as nucleic acids, proteins and cell wall components. A significant portion, as much as 70%, of the wall of Gram-negative bacteria is composed (as much as 70%), of lipopolysaccharides (termed pyrogens or endotoxins), peptidoglycans and proteins.

The effective removal of pyrogens from bacterial lysates is a key requirement in the production of bacteriophage preparations for dedicated bacterial infection therapies. Endotoxins are strong stimulants of the immune system by inducing the production of interleukins, TNF, NO, etc.

Endotoxin isolation or removal procedures are based on organic solvent extraction, such as an aqueous phenol solution Westphal O., Lueritz O., Bister F. Uber die Extraktion von Bakterien mit Phenol/wasser. Z. Naturforsch. 7: 148-155, 1952), mixtures of acids and aliphatic amines (Patent application Publicatiom US 2007/0020292A1), or extractive and chromatographic methods (Patent Application publication US 2007/0031447 AI). Purification of biological preparations of endotoxins is performed using metal ion-protein interactions (patent US 6,942,802 B2 Sep. 13, 2005; WO02083710A1; WO04003215A1), via the alcohol precipitation of endotoxins and with bivalent counterions (US 5039610). The use of bivalent ions in combination with alcohols, gels and detergents is the subject of many patents, such as EPO 407037B1. Crab haemolymph proteins have been used for endotoxin removal (US 5760177). Many column chromatography methods have been descrigel. They make use of lipopolysaccharide affinity for alkaline haptens such as polymyxine (Petsch D, Beeskow TC, Anspach FB, Deckwer WD, (1997) Membrane adsorbers for selective removal of bacterial endotoxin. J. Chromatogr B Biomed Sci Appl. 693(1):79-91), calcium silicate (Hang JP, Wang Q, Smith TR, Hurst WE, Sulpizio T, (2005) Endotoxin removal using a synthetic adsorbent of crystalline calcium silicate hydrate. Biotechnol Prago 21(4):1220-5), synthetic polymers (Hirayama Ch, Sakata M, (2002) Chromatographic removal of endotoxin from protein solutions by polymer particles. Journal oj Chramatography B, 781:419-432*)* or polyanionic gels (Boratyński J, Syper D, Weber-Dqbrowska B, 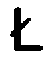 usiak-Szelachowska M, Pozniak G, Górski A. Preparation of endotoxin-free bacteriophages Cell Mol Biol Lett. 2004; 9(2):253-9).

There is still a need for methods of purifying bacteriophage lysates, particularly of endotoxins, which could be used for the industrial production of bacteriophage preparations meant for use in the treatment of bacterial infections which occur in humans. Despite the multiple aforementioned methods of purifying bacteriophage lysates, they fail to sufficiently fulfill the requirements for industrial production methods of preparations for the above mentioned uses.

Unexpectedly, a method fulfilling the above mentioned requirements has been proposed within the present invention.

The subject the present invention is a method of producing bacteriophages, characterised in that:
a) a bacterial host strain is cultured in a medium appropriate to it, the culture is inoculated with a bacteriophage strain and a bacteriophage lysate is obtained,
b) the bacteriophage lysate is purified using affinity chromatography, and
c) the purified bacteriophage preparation is obtained from the resulting eluate,
wherein the bacteriophage lacks a gene encoding a structural protein and the bacterial host cell contains a sequence that encodes the structural protein lacking in the bacteriophage as a fusion protein with HisTag or GST and has an affinity for a chromatography gel of stage b).

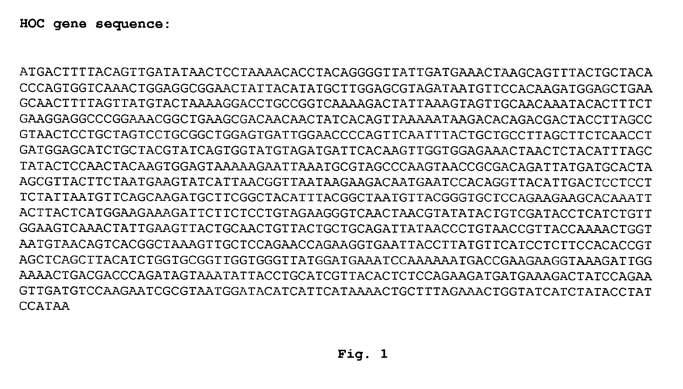

An example polypeptide from a phage structural protein is encoded by a sequence encoding the HOC protein shown in Figure 1. Preferably, during stage a), the bacterial host culture is maintained in a culture broth with a pH of about 7.2 which contains a meat extract, an enzymatic lysate of casein, yeast hydrolysate, peptone and NaCl. Preferably, during stage a), the bacterial host strain used is a bacterial strain sensitive to the phage being amplified.

During stage a), the culture is inoculated with a bacteriophage strain lacking the gene encoding the structural phage protein which is a part of the fusion protein encoded by the sequence contained in the bacteriophage host. Preferably, during stage a), the resulting phage lysate is filter-sterilised through a sterilising filter with 0.22 µm pores. Affinity chromatography is an established, highly efficient purification strategy for individual proteins of various origins. Unexpectedly, the use of such a method in the method according to the present invention, for the isolation of complete bacteriophage capsids, constituting complex and extensive spatial protein structures made it possible to retain the antibacterial properties of the isolated bacteriophages, despite the fact that their capsids have been extensively modified to enable the use of affinity chromatography. In the example embodiment, we made use of the phage display technique for introducing binding motifs into the phage capsid, and then we based on binding such modified bacteriophages to the affinity gel appropriate for the selected binding motif.

### Example 1

The procedure bases on the preparation and use of a parental bacteriophage strain, in which one of the non-essential structural genes (genes encoding facultative capsid proteins, whose absence does not inhibit phage activity) is deleted or damaged. In bacteriophage T4 this may be a defective *hoc* or *soc* gene. As the host for the defective T4 bacteriophage we used expressive strains of *Escherichia coli* transformed with expressive plasmids containing a proper *hoc* gene fused with a sequence encoding the selected binding motif. In such a culture, a bacteriophage defective in the selected capsid protein is able to supplement this absence with the Hoc protein with binding motif simultaneously expressed in the bacterial cell from the expression plasmid. This results in stable capsid structures containing the recombinant protein, and thus containing motifs with a strong affinity for the binding gels and expressing them on the capsid surface.

Two alternate means may be used to release the bacteriophages from the gel: (i) competitive elution, i.e. flushing by compounds capable of interacting with the binding motifs of the capsids and/or the binding gel (glutathione, imidazole), or (ii) proteolytic elution with the aid of a protease that recognises rare amino-acid motifs. The second strategy requires the introduction of sequences recognised by an appropriate protease at the design stage of the expression plasmid construct meant for the production of the recombinant protein in the cell. In the case of proteolytic elution, the bacteriophage capsid devoided of binding motifs.

A detailed example of an embodiment of the method according to the present invention is as follows.

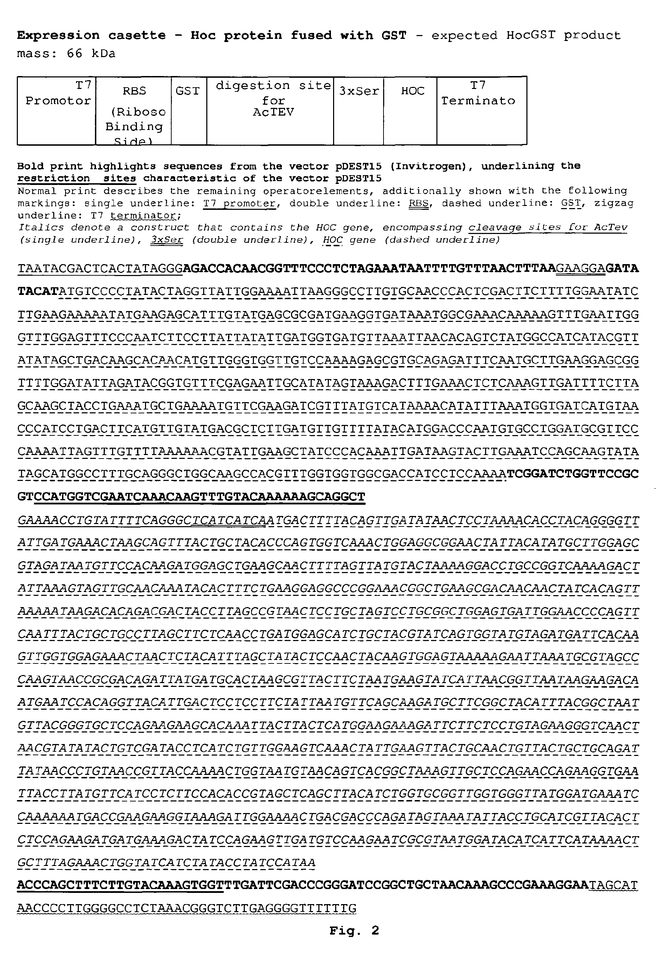

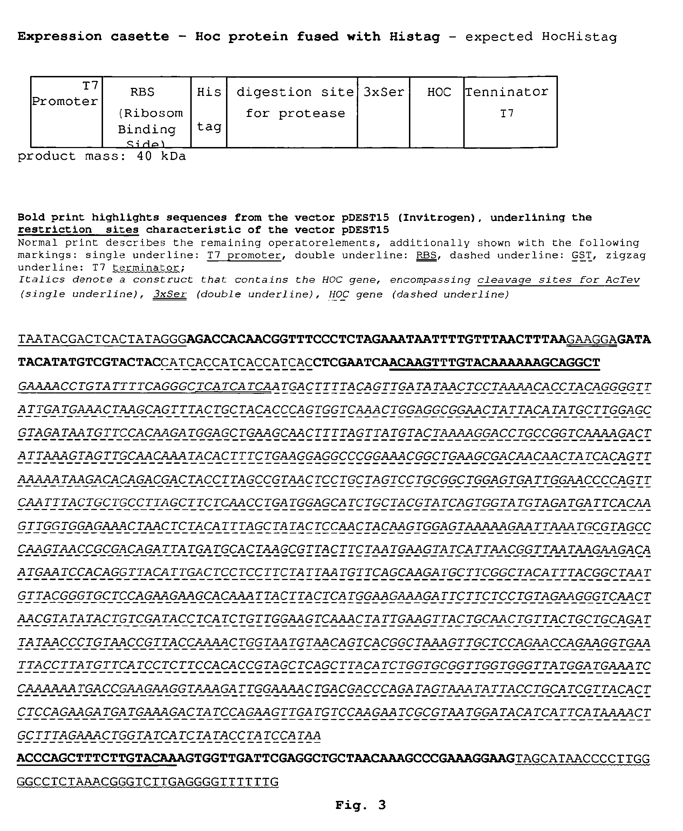

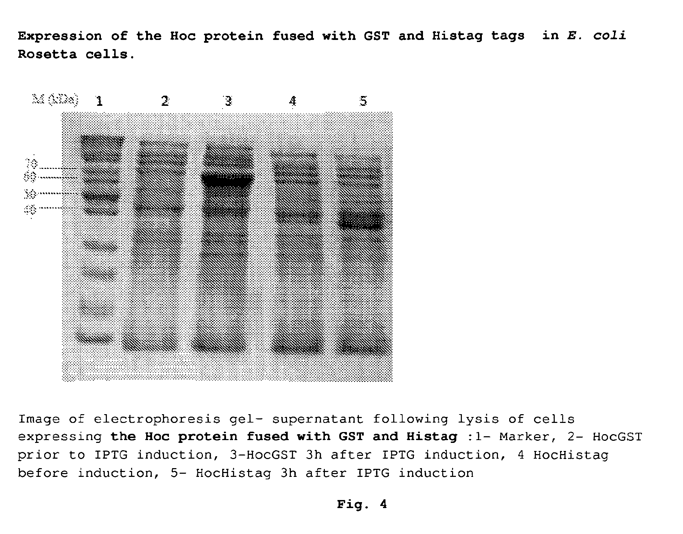

Bacterial host cells were obtained using expressive strains of *Escherichia coli* which were transformed with expression plasmids containing the correct *hoc* gene fused to a sequence encoding a selected binding motif. In the example embodiment we made use of the plasmid pDEST15 (Invitrogen), which contained an expression cassette that made it possible to obtain a protein containing Hoc fused with GST (Figure 2) or an expression cassette encoding a protein containing Hoc fused with Histag (Figure 3). The efficacy of the transformation of *E.coli Rosetta* cells was evaluated by observing the expression of the Hoc protein fused with the GST or Histag tags (Figure 4). Recombined host cells were cultured at a temperature of 37°C to an OD₆₀₀ of 0.7 LB medium (LB-Broth, high salt) containing: enzymatic casein hydrolysate 10g/l, yeast extract 5g/l, sodium chloride 10 g/l, pH 7.5. Next the cells were transferred into fresh LB (at a ratio of 1:100 in relation to the LB) with an addition of 0.0025 M IPTG and 1:100 in relation to the lysate of the HAP1 phage (~3x10⁹ pfu/ml). The induction of the expression and infection were thus simultaneous. The infected cells were cultured at 37°C at 160 RPM for 8 hours.

In the example embodiment of the present invention being described, the recombined *Escherichia coli* bacteria were infected with defective bacteriophage T4 (strain HAP1 deposited in the PCM repository as F/00028 and disclosed previously in the patent application PL355355, for which patent PL 195 815 has been granted), at the same time inducing the production of the Hoc protein. The phage lysis was performed concurrently, along with the induction of the expression of the recombined protein.

The phage lysate was filtered, whereafter it was incubated with an appropriate agarose gel: glutathione sepharose or agarose with metallic ions that form complexes with the imidazole residues of the histidines (i.e. agar NiNTA). The lysates were incubated with the appropriate gel overnight at 4°C wit h gentle rocking. After removing the unbound fraction, the gels were rinsed with 3 I of the following buffer: 50 mM Na₂HPO₄, 300 mM NaCl, pH 7.5.

Next, we used a typical purification procedure for affinity chromatography, the gel was rinsed, and the bacteriophages were eluted from the gel proteolytically.

For GST, we used two elution methods:
competitive elution with 40mM reduced glutathione;
   - elution buffer: 40mM reduced glutathione, 50mM Tris, pH 8.0. Before the collection of eachfraction, the gel was incubated in the buffer for 20 minutes;
proteolytic elution was performed using the AcTEV protease;
   - protease buffer: 50mM Tris, 0.5mM EDTA, 1 mM DTT, pH 8.0. The enzyme was added at 1µl per 1 ml gel (activity 10U/µl). Proteolysis was performed over 7 days.
From NiNTA gels, the phages were flushed out with imidazole in a 100-500mM gradient
   - elution buffer: 100-500mM imidazole (depending on the fraction), 50 mM Na2HP04,300 mM NaCl, pH 7.5 The rinsing and elution were performed at room temperature.

### Methods of analyzing the results

The specificity of bacteriophages modified in relation to the gel was estimated on the basis of a comparison of the elution profiles of modified T4 phages, unmodified T4 phages and T4 phages modified with a tag incompatible with the gel used. The elution profile was determined by evaluating the phage titer in individual fractions. The effectiveness of the purification was evaluated by determining the endotoxin level in the eluted (or released) fractions.

### Results

Te results obtained are shown in the attached Figures and Tables.

The graphs shown in the Figures contain comparisons of individual elution profiles. In the case o comparative experiments meant to show the specificity of the phages for a gel, the initial titres of the control preparation and potentially specific preparation are the same. The same volumes of lysates were incubated with the same amount of gel, rinsed and eluted in identical conditions.

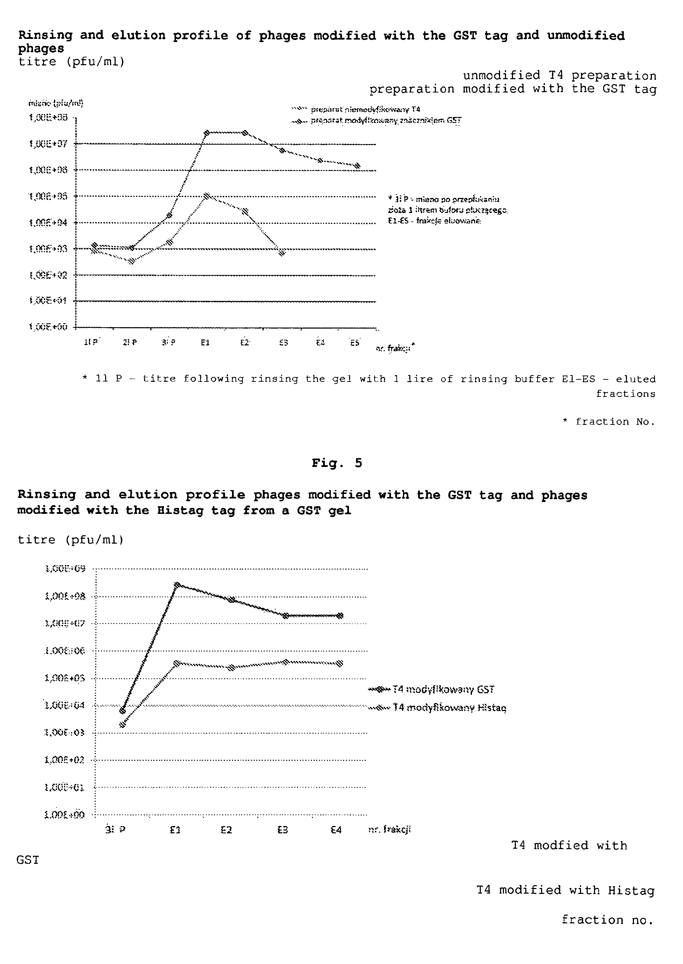

Fig. 5 shows a comparison of the elution profiles of phages modified with the GST tag and unmodified ones. The affinity of the modified preparation for the gel is almost 1000-fold that of the unmodified phage, which shows its specificity towards GST.

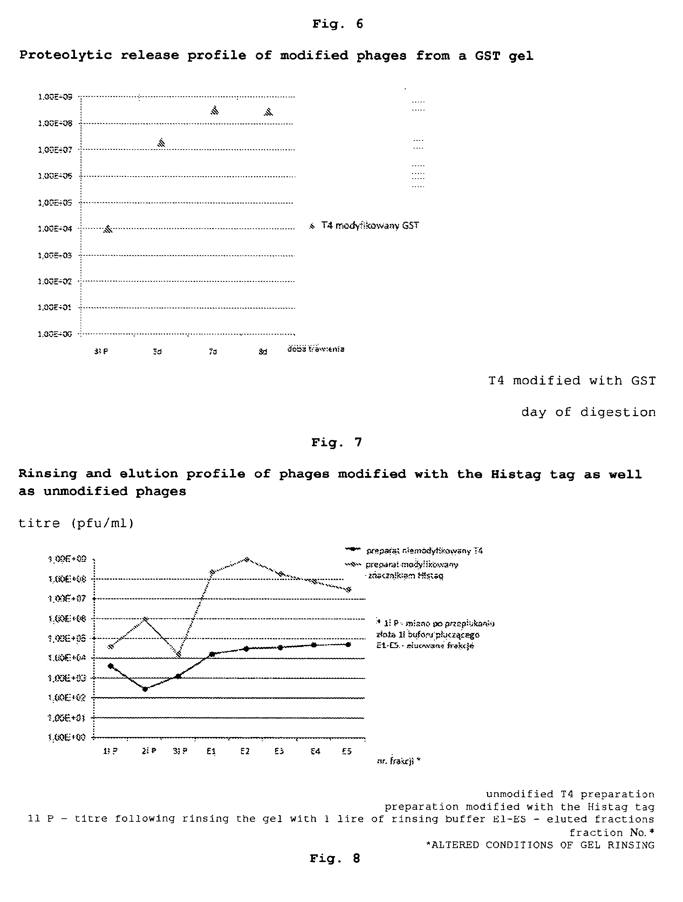

Fig. 6 shows a comparison an elution profile of phages modified with a GST tag as well as of phages modified with a Histag tag. The affinity of the modified preparation for the gel is nearly 1000-fold that of the preparation modified with the incompatible tag, which shows its specificity towards GST.

Fig. 7 shows a proteolytic release profile of modified T4 phages from a GST column.

Fig. 8 shows a comparison an elution profile of phages modified with a Histag tag as well as unmodified ones. The affinity of the modified preparation for the gel is almost 100000-fold that of the unmodified phage, which shows its specificity towards NiNTA.

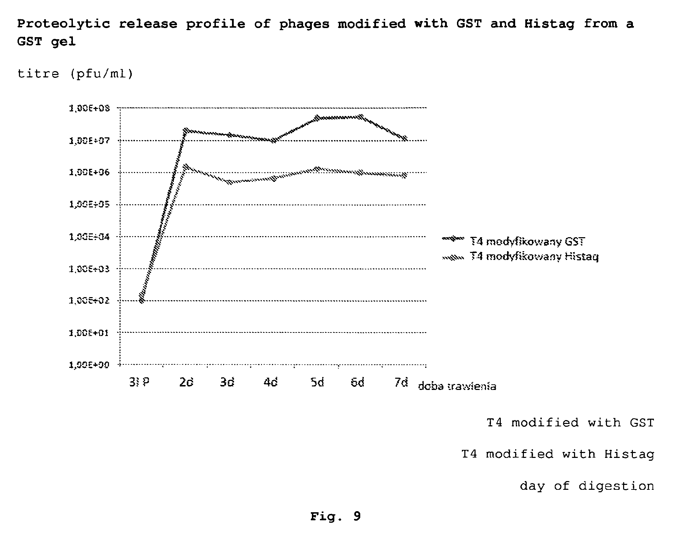

Fig. 9 shows a comparison of the proteolytic release profile of phages modified with a GST tag as well as of phages modified with a Histag tag from a GST column. The affinity of the modified preparation for the gel i nearly 100-fold that of the preparation modified with the incompatible tag, which shows its specificity for GST.

**Tab.1. The table shows the resulting endotoxin values of purified phage preparations and their corresponding titres.**

| Phage preparation | Titre(pfu/mi) | Endotoxin level (EU/ml) |
|---|---|---|
| T4 modified with GST | 1,3 x 10 | 186 |
| T4 modified with NiNTA | 4,7 x 10⁸ | 253 |
| T4 modified with GST- | 4,7 x 10^{H} | 363 |
| Proteolysis | | |
| T4 modified with GST- | 1 x 10 | 11 |
| Proteolysis * | | |
| T4 modified with HisTaq* | 1 x 10 | 16 |
| T4 modified with GST* | 7 x 10⁸ | 13 |

### Conclusions

The proposed method facilitates the single-stage and effective purification of phage preparations, makes it possible to retain the antibacterial activity of bacteriophages, both in the case of the strategy based on the displacement of bound bacteriophages from the gel, as well as proteolytic elution. It does not require additional steps for removing proteinaceous and non-proteinaceous contaminants (including LPS). The modification of phage capsid proteins with appropriate binding motifs can also facilitate the purification of other bacteriophage strains of using the affinity chromatography technique.

## Claims

1. A method of producing bacteriophages, **characterised in that**:
a) a bacterial host strain is cultured in a medium appropriate to it, the culture is inoculated with a bacteriophage strain and a bacteriophage lysate is obtained,
b) the bacteriophage lysate is purified using affinity chromatography, and
c) the purified bacteriophage preparation is obtained from the resulting eluate,
wherein the bacteriophage lacks a gene encoding a structural protein and the bacterial host cell contains a sequence that encodes the structural protein lacking in the bacteriophage as a fusion protein with HisTag or GST and has an affinity for a chromatography gel of stage b).

2. A method according to Claim 1, **characterised in that** during stage a), the bacterial host cell culture is conducted in a culture broth with a pH of about 7.2 containing a meat extract, enzymatic casein hydrolysate, yeast hydrolysate, peptone and NaCl.

3. A method according to Claim 1, **characterised in that** during stage a) the bacterial host strain used is a bacterial strain sensitive to the lytic activity of the amplified bacteriophage.

4. A method according to Claim 1, **characterised in that** during stage a) the resulting phage lysate is sterilised through filtration via a 0.22 µm sterilising filter.

## Patentansprüche

1. Verfahren zur Herstellung von Bakteriophagen, **dadurch gekennzeichnet, dass**:
a) ein bakterieller Wirtsstamm in einem für ihn geeigneten Medium kultiviert wird, die Kultur mit einem Bakteriophagenstamm beimpft wird und ein Bakteriophagenlysat erhalten wird,
b) das Bakteriophagenlysat unter der Verwendung von Affinitätchromatographie gereinigt wird, und
c) die gereinigte Bakteriophagenpräparation aus dem sich ergebenden Eluat erhalten wird,
wobei dem Bakteriophagen ein Gen fehlt, das für ein strukturelles Protein kodiert, und die bakterielle Wirtszelle eine Sequenz enthält, die für das strukturelle Protein kodiert, das in dem Bacteriophagen fehlt, als ein Fusionsprotein mit His-Tag oder GST und eine Affinität für ein Chromatographie-Gel der Phase b) aufweist.

2. Verfahren nach Anspruch 1, **dadurch gekennzeichnet, dass** während der Phase a) die bakterielle Wirtszellkultur in einem Kulturmedium mit einem pH von ungefähr 7,2 durchgeführt wird, das einen Fleischextrakt, enzymatisches Caseinhydrolysat, Hefehydrolysat, Pepton und NaCl enthält.

3. Verfahren nach Anspruch 1, **dadurch gekennzeichnet, dass** während der Phase a) der verwendete bakterielle Wirtszellstamm ein bakterieller Stamm ist, der gegenüber der lytischen Aktivität des amplifizierten Bacteriophagen sensitiv ist.

4. Verfahren nach Anspruch 1, **dadurch gekennzeichnet, dass** während der Phase a) das resultierende Phagenlysat durch Filtration durch einen 0,22 µm sterilisierenden Filter sterilisiert wird.

## Revendications

1. Procédé de production de bactériophages, **caractérisé en ce que** :
a) une souche hôte bactérienne est mise en culture dans un milieu qui lui est approprié, la culture se voit inoculer une souche de bactériophage et un lysat de bactériophage est obtenu,
b) le lysat de bactériophage est purifié en utilisant la chromatographie par affinité, et
c) la préparation du bactériophage purifié est obtenue à partir du produit d'élution résultant,
dans lequel le bactériophage n'a pas de gène codant pour une protéine structurale et la cellule hôte bactérienne contient une séquence qui code pour la protéine structurale manquante dans le bactériophage en tant que protéine de fusion avec HisTag ou GST et a une affinité pour un gel de chromatographie de l'étape b).

2. Procédé selon la revendication 1, **caractérisé en ce que** pendant l'étape a) la culture de cellules hôtes bactériennes est conduite dans un bouillon de culture avec un pH d'environ 7,2 contenant un extrait de viande, un hydrolysat de caséine enzymatique, un hydrolysat de levure, de la peptone et du NaCl.

3. Procédé selon la revendication 1, **caractérisé en ce que** pendant l'étape a) la souche hôte bactérienne utilisée est une souche bactérienne sensible à l'activité lytique du bactériophage amplifié.

4. Procédé selon la revendication 1, **caractérisé en ce que** pendant l'étape a) le lysat de phage résultant est stérilisé par le biais d'une filtration via un filtre stérilisateur de 0,22 µm.


### SEQUENCE LISTING

<110> Instytut Immunologii i Terapii Do\234wiadczalnej PAN
<120> A method of producing bacteriophages
<130> PZ/1030/RW
<160> 3
<170> PatentIn version 3.3
<210> 1
   <211> 1131
   <212> DNA
   <213> artificial
<220>
   <223> gene HOC
<400> 1
   atgactttta cagttgatat aactcctaaa acacctacag gggttattga tgaaactaag      60
   cagtttactg ctacacccag tggtcaaact ggaggcggaa ctattacata tgcttggagc     120
   gtagataatg ttccacaaga tggagctgaa gcaactttta gttatgtact aaaaggacct     180
   gccggtcaaa agactattaa agtagttgca acaaatacac tttctgaagg aggcccggaa     240
   acggctgaag cgacaacaac tatcacagtt aaaaataaga cacagacgac taccttagcc     300
   gtaactcctg ctagtcctgc ggctggagtg attggaaccc cagttcaatt tactgctgcc     360
   ttagcttctc aacctgatgg agcatctgct acgtatcagt ggtatgtaga tgattcacaa     420
   gttggtggag aaactaactc tacatttagc tatactccaa ctacaagtgg agtaaaaaga     480
   attaaatgcg tagcccaagt aaccgcgaca gattatgatg cactaagcgt tacttctaat     540
   gaagtatcat taacggttaa taagaagaca atgaatccac aggttacatt gactcctcct     600
   tctattaatg ttcagcaaga tgcttcggct acatttacgg ctaatgttac gggtgctcca     660
   gaagaagcac aaattactta ctcatggaag aaagattctt ctcctgtaga agggtcaact     720
   aacgtatata ctgtcgatac ctcatctgtt ggaagtcaaa ctattgaagt tactgcaact     780
   gttactgctg cagattataa ccctgtaacc gttaccaaaa ctggtaatgt aacagtcacg     840
   gctaaagttg ctccagaacc agaaggtgaa ttaccttatg ttcatcctct tccacaccgt     900
   agctcagctt acatctggtg cggttggtgg gttatggatg aaatccaaaa aatgaccgaa     960
   gaaggtaaag attggaaaac tgacgaccca gatagtaaat attacctgca tcgttacact    1020
   ctccagaaga tgatgaaaga ctatccagaa gttgatgtcc aagaatcgcg taatggatac    1080
   atcattcata aaactgcttt agaaactggt atcatctata cctatccata a             1131
<210> 2
   <211> 2069
   <212> DNA
   <213> artificial
<220>
   <223> casette HOC+GST
<400> 2
   taatacgact cactataggg agaccacaac ggtttccctc tagaaataat tttgtttaac      60
   tttaagaagg agatatacat atgtccccta tactaggtta ttggaaaatt aagggccttg     120
   tgcaacccac tcgacttctt ttggaatatc ttgaagaaaa atatgaagag catttgtatg     180
   agcgcgatga aggtgataaa tggcgaaaca aaaagtttga attgggtttg gagtttccca     240
   atcttcctta ttatattgat ggtgatgtta aattaacaca gtctatggcc atcatacgtt     300
   atatagctga caagcacaac atgttgggtg gttgtccaaa agagcgtgca gagatttcaa     360
   tgcttgaagg agcggttttg gatattagat acggtgtttc gagaattgca tatagtaaag     420
   actttgaaac tctcaaagtt gattttctta gcaagctacc tgaaatgctg aaaatgttcg     480
   aagatcgttt atgtcataaa acatatttaa atggtgatca tgtaacccat cctgacttca     540
   tgttgtatga cgctcttgat gttgttttat acatggaccc aatgtgcctg gatgcgttcc     600
   caaaattagt ttgttttaaa aaacgtattg aagctatccc acaaattgat aagtacttga     660
   aatccagcaa gtatatagca tggcctttgc agggctggca agccacgttt ggtggtggcg     720
   accatcctcc aaaatcggat ctggttccgc gtccatggtc gaatcaaaca agtttgtaca     780
   aaaaagcagg ctgaaaacct gtattttcag ggctcatcat caatgacttt tacagttgat     840
   ataactccta aaacacctac aggggttatt gatgaaacta agcagtttac tgctacaccc     900
   agtggtcaaa ctggaggcgg aactattaca tatgcttgga gcgtagataa tgttccacaa     960
   gatggagctg aagcaacttt tagttatgta ctaaaaggac ctgccggtca aaagactatt    1020
   aaagtagttg caacaaatac actttctgaa ggaggcccgg aaacggctga agcgacaaca    1080
   actatcacag ttaaaaataa gacacagacg actaccttag ccgtaactcc tgctagtcct    1140
   gcggctggag tgattggaac cccagttcaa tttactgctg ccttagcttc tcaacctgat    1200
   ggagcatctg ctacgtatca gtggtatgta gatgattcac aagttggtgg agaaactaac    1260
   tctacattta gctatactcc aactacaagt ggagtaaaaa gaattaaatg cgtagcccaa    1320
   gtaaccgcga cagattatga tgcactaagc gttacttcta atgaagtatc attaacggtt    1380
   aataagaaga caatgaatcc acaggttaca ttgactcctc cttctattaa tgttcagcaa    1440
   gatgcttcgg ctacatttac ggctaatgtt acgggtgctc cagaagaagc acaaattact    1500
   tactcatgga agaaagattc ttctcctgta gaagggtcaa ctaacgtata tactgtcgat    1560
   acctcatctg ttggaagtca aactattgaa gttactgcaa ctgttactgc tgcagattat    1620
   aaccctgtaa ccgttaccaa aactggtaat gtaacagtca cggctaaagt tgctccagaa    1680
   ccagaaggtg aattacctta tgttcatcct cttccacacc gtagctcagc ttacatctgg    1740
   tgcggttggt gggttatgga tgaaatccaa aaaatgaccg aagaaggtaa agattggaaa    1800
   actgacgacc cagatagtaa atattacctg catcgttaca ctctccagaa gatgatgaaa    1860
   gactatccag aagttgatgt ccaagaatcg cgtaatggat acatcattca taaaactgct   1920
   ttagaaactg gtatcatcta tacctatcca taaacccagc tttcttgtac aaagtggttt   1980
   gattcgaccc gggatccggc tgctaacaaa gcccgaaagg aatagcataa ccccttgggg   2040
   cctctaaacg ggtcttgagg ggttttttg                                      2069
<210> 3
   <211> 1411
   <212> DNA
   <213> artificial
<220>
   <223> cassette HOC + HisTag
<400> 3
   taatacgact cactataggg agaccacaac ggtttccctc tagaaataat tttgtttaac     60
   tttaagaagg agatatacat atgtcgtact accatcacca tcaccatcac ctcgaatcaa    120
   caagtttgta caaaaaagca ggctgaaaac ctgtattttc agggctcatc atcaatgact    180
   tttacagttg atataactcc taaaacacct acaggggtta ttgatgaaac taagcagttt    240
   actgctacac ccagtggtca aactggaggc ggaactatta catatgcttg gagcgtagat    300
   aatgttccac aagatggagc tgaagcaact tttagttatg tactaaaagg acctgccggt    360
   caaaagacta ttaaagtagt tgcaacaaat acactttctg aaggaggccc ggaaacggct    420
   gaagcgacaa caactatcac agttaaaaat aagacacaga cgactacctt agccgtaact    480
   cctgctagtc ctgcggctgg agtgattgga accccagttc aatttactgc tgccttagct    540
   tctcaacctg atggagcatc tgctacgtat cagtggtatg tagatgattc acaagttggt    600
   ggagaaacta actctacatt tagctatact ccaactacaa gtggagtaaa aagaattaaa    660
   tgcgtagccc aagtaaccgc gacagattat gatgcactaa gcgttacttc taatgaagta    720
   tcattaacgg ttaataagaa gacaatgaat ccacaggtta cattgactcc tccttctatt    780
   aatgttcagc aagatgcttc ggctacattt acggctaatg ttacgggtgc tccagaagaa    840
   gcacaaatta cttactcatg gaagaaagat tcttctcctg tagaagggtc aactaacgta    900
   tatactgtcg atacctcatc tgttggaagt caaactattg aagttactgc aactgttact    960
   gctgcagatt ataaccctgt aaccgttacc aaaactggta atgtaacagt cacggctaaa   1020
   gttgctccag aaccagaagg tgaattacct tatgttcatc ctcttccaca ccgtagctca   1080
   gcttacatct ggtgcggttg gtgggttatg gatgaaatcc aaaaaatgac cgaagaaggt   1140
   aaagattgga aaactgacga cccagatagt aaatattacc tgcatcgtta cactctccag   1200
   aagatgatga aagactatcc agaagttgat gtccaagaat cgcgtaatgg atacatcatt   1260
   cataaaactg ctttagaaac tggtatcatc tatacctatc cataaaccca gctttcttgt   1320
   acaaagtggt tgattcgagg ctgctaacaa agcccgaaag gaagtagcat aaccccttgg   1380
   ggcctctaaa cgggtcttga ggggtttttt g                                  1411